# EUROPEAN PATENT SPECIFICATION

(11) **EP 2 612 596 B1**
(45) Date of publication and mention of the grant of the patent: **13.09.2017**
(21) Application number: 13150331.0
(22) Date of filing: 04.01.2013
(51) Int. Cl.: A61B 8/00, A61B 8/08, G06F 19/26

(54) **Method and apparatus for measuring biometrics of object**
Verfahren und Vorrichtung zur Messung der Biometrik eines Objekts
Procédé et appareil de mesure biométrique d'objet

(30) Priority: 04.01.2012 KR 20120001150
(43) Date of publication of application: 10.07.2013
(73) Proprietor: Samsung Electronics Co., Ltd., Gyeonggi-do 443-742 (KR)
(72) Inventor: Jung, Hae-kyung, Seoul (KR); Yoon, Hee-chul, Seoul (KR); Lee, Hyun-taek, Seoul (KR); Kim, Yong-je, Gyeonggi-do (KR); Kim, Jae-hyun, Seoul (KR); Eom, Myung-jin, Seoul (KR)
(74) Representative: Appleyard Lees IP LLP

(56) References cited:
- EP-A1- 0 916 308
- EP-A1- 2 387 949
- US-A- 5 605 155
- US-A1- 2002 102 023
- YU-BU LEE ET AL: "Robust border enhancement and detection for measurement of fetal nuchal translucency in ultrasound images", MEDICAL & BIOLOGICAL ENGINEERING & COMPUTING, SPRINGER, BERLIN, DE, vol. 45, no. 11, 27 July 2007 (2007-07-27) , pages 1143-1152, XP019864740, ISSN: 1741-0444, DOI: 10.1007/S11517-007-0225-7

## Description

### CROSS-REFERENCE TO RELATED PATENT APPLICATION

This application claims the benefit of Korean Patent Application No.10-2012-0001150, filed on January 4, 2012, in the Korean Intellectual Property Office.

### BACKGROUND OF THE INVENTION

### 1. Field of the Invention

One or more aspects of the present invention relate to a method and apparatus for automatically measuring biometrics of an object from e.g. an ultrasound image, or a CT image, or an MRI image or an X-ray image or the like, etc, of the object.

### 2. Description of the Related Art

Ultrasound systems have noninvasive and nondestructive characteristics and thus have largely been used in the medical field to obtain information about the inside of an object. The ultrasound systems provide a doctor with a high-resolution image of the inside of an object in real time without performing a surgical operation on the object. Thus, the ultrasound systems have drawn much attention in the medical field.

Ultrasound images have been used for early diagnosis to determine whether a fetus has a defect in its chromosome or nervous system, e.g., Down's syndrome. In order for a diagnostician to accurately measure biometrics of the fetus and diagnose a state of the fetus by determining the location of the fetus with the naked eyes, an image of a mid-sagittal plane of the fetus is detected and a fetal crown-rump length (CRL), a nuchal translucency (NT), and an intracranial translucency (IT) of the fetus are measured based on the image.

Although biometrics, such as the CRL, NT, and IT, are individually measured and output, a relative difference between either the NT or the IT and the CRL, i.e., a value calculated based on at least two biometrics is used to diagnose the state of the fetus. Thus, there is a need to automatically provide a user with a value calculated based on a result of integrating biometrics, such as the CRL, the NT, and IT, and a result of diagnosing the fetus based on the calculated value so that the user may easily diagnose and determine the state of the fetus.

EP-A-2387949 discloses a three-dimensional (3D) ultrasound system and a method for operating the 3D ultrasound system. The 3D ultrasound system includes a display unit to form an image with respect to an object in the body and to display the image on a screen, and a control unit to generate a figure template corresponding to the image, to adjust the generated figure template based on a reference location of the image, and to superimpose the adjusted figure template on the image and display the resulting combination.

US patent 5,605,155 discloses an ultrasound system that automatically measures fetal head size from ultrasound images. An ultrasound image of the fetal head is detected. A radial maxima point is identified on each of a plurality of radii extending from a substantially common vertex point within the fetal head image. Each radial maxima point corresponds to an ultrasound sample along its corresponding radius, and has a maximum ultrasound echo strength. A first curve is defined from the radial maxima points. The remaining unfiltered radial maxima points are fit to a second curve, and the second curve is the detected curved boundary. The detected curve boundary is modified to define an initial fetal head boundary. An inner fetal head boundary and outer fetal head boundary are derived from the initial fetal head boundary and a predetermined fetal skull thickness, and fetal head size is computed from the inner fetal head boundary and the outer fetal head boundary.

EP-A-0916308 discloses an arrangement to decrease the amount of operation required for an operator to move a cursor and in order to allow the operator to distinctly view deviation of CRL (Crown Rump Length) of a fetus from a normal value, when an ultrasonic image of a fetus F is displayed and a first cursor C1 is positioned at the head (or the rump) of the fetus F, a second cursor C2 is initially displayed at a position which corresponds to a normal CRL obtained from GA (Gestation Age) estimated from the last menstruation date. When the second cursor C2 is moved and positioned at the rump (or the head) of the fetus F, the measured CRL and GA estimated therefrom are displayed.

### SUMMARY OF THE INVENTION

One or more aspects of the present invention provide a method and apparatus for automatically measuring biometrics of an object by using an image (e.g. ultrasound image) of the object. According to the present invention there is provided an apparatus and method as set forth in the appended claims. Other features of the invention will be apparent from the dependent claims, and the description which follows.

According to an aspect of the present invention, there is provided a method of measuring biometrics of an object, the method including receiving an image of the object, modeling the object such that at least one part of the object is identified, and measuring biometrics of the object, based on a result of modeling the object.

The modeling of the object may include displaying a result of modeling the at least one part of the object in an oval shape including a circular shape, and modifying the result of modeling the object, based on a user input signal. Modeling of the object may be estimated and performed again by controlling the figure obtained by modeling the object such that a central axis on a head or body may be moved to a side.

The measuring of the biometrics of the object includes determining whether the measured biometrics fall within a normal range, modeling the object again by estimating a model of the object corresponding to a case where the biometrics fall within the normal range when it is determined that the measured biometrics do not fall within the normal range, and measuring the biometrics of the object again, based on the estimated modeling result.

The measuring of the biometrics of the object may include detecting a region-of-interest (ROI) for measuring the biometrics, based on the result of modeling the object; and measuring the biometrics in the ROI.

The detecting of the ROI may include displaying the detected ROI to be differentiated from the other parts of the object, displaying a region for measuring the biometrics, in the ROI, and modifying the ROI or the region for measuring the biometrics, according to a user input signal.

The modeling of the object includes modeling the object such that a body and head of the object are identified.

After the modeling of the object, the method further includes detecting at least one characteristic point on the head of the object, setting a central axis by using the at least one characteristic points and then displaying the central axis, measuring an angle between the body and head of the object with respect to the central axis, determining whether the angle falls within a normal range, and measuring a crown-rump length (CRL) of the object, based on a result of the determining.

The measuring of the CRL includes measuring the CRL of the object based on the result of modeling the object when the angle falls within the normal range, estimating a model of the object corresponding to a case where the angle falls within the normal range when the measured angle does not fall within the normal range and then measuring the CRL of the object, based on the estimated modeling result, and displaying the estimated modeling result and the measured CRL. Modeling of the object may be estimated and performed again by controlling the figure obtained by modeling the object such that a central axis on a head or body may be moved to a side.

The measuring of the biometrics may include measuring a crown-rump length (CRL) of the object, measuring at least one among a nuchal translucency (NT) and an intracranial translucency (IT) of the object, calculating a relative difference between the CRL and either the NT or the IT, and displaying the measured CRL, NT, and IT.

According to another aspect of the present invention, there is provided a terminal apparatus for measuring biometrics of an object, the terminal apparatus including a storage unit for storing an image of the object, and a control unit. The control unit includes a modeling module for modeling the object such that at least one part of the object is identified in the image of the object; and a measuring module for measuring biometrics of the object, based on a result of modeling the object.

The terminal apparatus may further include an input unit for receiving a user input. The modeling module may modify the result of modeling the object, based on a user input signal.

The storage unit may store biometrics data including information about a normal range of at least one of the biometrics. If the measured biometrics do not fall within the normal range, the modeling module may model the object again by estimating a model of the object corresponding to a case where the biometrics fall within the normal range. If the measured biometrics do not fall within the normal range, the measuring module may measure the biometrics of the object again, based on the estimated modeling result.

The control unit may further include a calculating module for calculating an error rate between a result of measuring the biometrics again and the biometrics measured using the result of modeling the object.

The measuring module may detect a region-of-interest (ROI) for measuring the biometrics, based on the result of modeling the object, and measure the biometrics in the ROI. The terminal apparatus may further include an output unit for outputting the detected ROI to be differentiated from the other parts of the object, and output a region for measuring the biometrics in the ROI.

The terminal apparatus may further include an input unit for receiving a user input. The measuring module may modify the ROI according to a user input signal.

The modeling module models the object such that a body and head of the object are identified.

The modeling module detects at least one characteristic point on the head of the object, and set a central axis by using the at least one characteristic point.

The storage unit stores biometrics data including information about a normal range of at least one of the biometrics. The measuring module measures an angle between the body and head of the object, determines whether the angle falls within a normal range, and measure a crown-rump length (CRL) of the object, based on a result of the determining. The terminal apparatus includes an output unit for outputting a result of estimating a result of modeling the object when the angle falls within a normal range and a result of measuring the CRL of the object.

If the angle falls within the normal range, the measuring module measures the CRL of the object based on the result of modeling the object. If the angle does not fall within the normal range, the measuring module estimates a model of the object corresponding to a case where the angle falls within the normal range, and measure the CRL of the object based on the estimated modeling result.

The measuring module may measure the CRL of the object, and measure at least one among a nuchal translucency (NT) and an intracranial translucency (IT) of the object. The control unit may further include a calculating module for calculating a relative difference between the CRL and either the NT or IT.

The control unit may provide a user interface via which after modeling the object or extracting a region for measuring the biometrics of the object is automatically performed, whether a result of modeling the object or the extracted region is set to be verified by a user, according to a user input signal.

The control unit may provide a user interface via which whether at least one among modeling the object, extracting a region for measuring the biometrics of the object, and estimating a result of modeling the object is to be performed automatically or manually, is set according to a user input signal.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of a terminal apparatus that measures biometrics of an object, according to a first embodiment of the present invention;
FIG. 2 is a block diagram of a terminal apparatus that measures biometrics of an object, according to a second embodiment of the present invention;
FIG. 3 is a flowchart illustrating a method of measuring biometrics of an object, according to the first embodiment of the present invention;
FIG. 4 is a flowchart illustrating a method of measuring biometrics of an object, according to the second embodiment of the present invention;
FIG. 5 is a flowchart illustrating a method of measuring a crown-rump length (CRL) of an object, according to a third embodiment of the present invention;
FIG. 6 is a flowchart illustrating a method of measuring a nuchal translucency (NT) or an intracranial translucency (IT) of an object, according to a fourth embodiment of the present invention;
FIG. 7 is a block diagram of a system that measures biometrics of an object, according to an embodiment of the present invention;
FIG. 8 is a block diagram of a service apparatus included in a system that measures biometrics of an object, according to a fifth embodiment of the present invention;
FIG. 9 is a block diagram of a service apparatus included in a system that measures biometrics of an object, according to a sixth embodiment of the present invention;
FIG. 10 is a flowchart illustrating a method of measuring biometrics of an object, according to the fifth embodiment of the present invention;
FIG. 11 is a flowchart illustrating a method of measuring biometrics of an object, according to the sixth embodiment of the present invention;
FIG. 12 is a flowchart illustrating a method of measuring a CRL of an object, according to a seventh embodiment of the present invention;
FIG. 13 is a flowchart illustrating a method of measuring an NT or an IT of an object, according to an eighth embodiment of the present invention;
FIGS. 14A and 14B illustrate examples of an ultrasound image of an object transmitted to a terminal apparatus or a service apparatus according to an embodiment of the present invention;
FIGS. 15A to 15C illustrate images each showing a result of modeling an object and a result of measuring a CRL, IT, and NT of the object, according to embodiments of the present invention; and
FIG. 16 illustrates a screen image on which whether an object is to be modeled, whether biometrics of the object are to be automatically measured, and whether a user will verify the measured biometrics after the measurement of the biometrics are set, according to an embodiment of the present invention.

### DETAILED DESCRIPTION

Hereinafter, exemplary embodiments of the present invention will be described more fully with reference to the accompanying drawings. In the following description, well-known functions or constructions are not described in detail if it is determined that they would obscure the invention due to unnecessary detail. In the drawings, like reference numerals denote like elements.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein. Although a few embodiments of the present invention have been shown and described, it would be appreciated by those of ordinary skill in the art that changes may be made in these exemplary embodiments without departing from the principles of the invention, the scope of which is defined in the claims and their equivalents.

As used herein, 'at least one of,' when preceding a list of elements, modify the entire list of elements and do not modify the individual elements of the list.

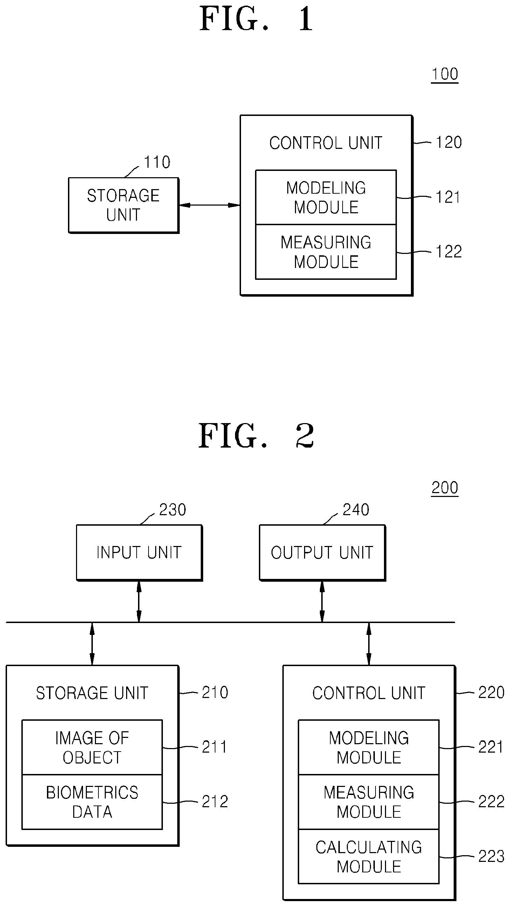

FIG. 1 is a block diagram of a terminal apparatus 100 that measures biometrics of an object, according to a first embodiment of the present invention. The terminal apparatus 100 of FIG. 1 may correspond to a terminal apparatus 200 of FIG. 2 which will be described below.

In the present invention, biometrics may be understood as including length information of a human body, for example, a crown-rump length (CRL), an intracranial translucency (IT), and a nuchal translucency (NT) of a fetus. According to the present invention, a state of an object may be diagnosed by measuring biometrics of the object, based on an image of the object.

Referring to FIG. 1, the terminal apparatus 100 according to the first embodiment of the present invention may include a storage unit 110 and a control unit 120.

The terminal apparatus 100 may be included as an element of an image analysis apparatus included in a medical image diagnosis apparatus, e.g., an X-ray apparatus, an ultrasound apparatus, a computed tomography (CT) apparatus, or magnetic resonance imaging (MRI) apparatus. Otherwise, the terminal apparatus 100 may be any of various apparatuses that a user uses, e.g., a personal computer (PC), a notebook computer, a mobile phone, a tablet PC, a navigation system, a smart phone, a personal digital assistant (PDA), a smart TV, a portable multimedia player (PMP), and a digital broadcasting receiver. In addition, the terminal apparatus 100 should be understood as a concept including all other apparatuses that are currently developed and placed on the market or that are to be developed in the future.

According to the first embodiment, the storage unit 110 stores data or a program for operating the terminal apparatus 100. Basically, the storage unit 110 may store an operating system (OS) of the terminal apparatus 100, at least one application program, and an image of the object. In this case, the image of the object may include an internal or external image of the object for measuring biometrics of the object, such as an ultrasound image, an MRI image, a CT image, or an X-ray image. The storage unit 110 may be any of various storage media, e.g., a random access memory (RAM), a read-only memory (ROM), a hard disk drive (HDD), a flash memory, a compact disc (CD)-ROM, and a digital versatile disc (DVD).

The control unit 120 controls overall operations of the terminal apparatus 100. Basically, the control unit 120 operates based on the OS stored in the storage unit 110 to build a basic platform environment of the terminal apparatus 100, and runs an application program to provide a desired function according to a user's selection.

Specifically, the control unit 120 may control such that an image of the object is received from an external device (not shown) or the storage unit 110, the object is modeled to identify respective parts thereof based on the image of the object, biometrics of the object are measured based on a result of modeling the object, and the measured biometrics and the result of modeling the object are then output to an external display unit (not shown) or an output unit (not shown) included in the terminal apparatus 100.

According to the first embodiment, the control unit 120 may include a modeling module 121 and a measuring module 122.

The modeling module 121 models the object such that the respective parts of the object may be identified, based on the image of the object. The object may be modeled in an oval shape including a circular shape, but is not limited thereto. If the object is a fetus, the head and body of the fetus may be modeled in a circular or oval shape to be differentiated from each other and a result of modeling the object may be output via an output unit.

The measuring module 122 measures biometrics of the object based on the result of modeling the object when the modeling module 121 models the object such that the respective parts of the object are identified. If the object is a fetus, then a central axis may be set on the circular or oval shape with which the object is modeled, based on characteristic points of the head and body of the fetus. The CRL, NT, and IT biometrics of the fetus may be measured based on the set central axis.

FIG. 2 is a block diagram of a terminal apparatus 200 that measures biometrics of an object, according to a second embodiment of the present invention. The terminal apparatus 200 of FIG. 2 may correspond to the terminal apparatus 100 of FIG. 1.

Referring to FIG. 2, the terminal apparatus 200 according to the second embodiment may include a storage unit 210, a control unit 220, an input unit 230, and an output unit 240. The storage unit 210 and the control unit 220 correspond to the storage unit 110 and the control unit 120, respectively, and are not described again here.

According to the second embodiment, there is provided a method of measuring biometrics, which is capable of increasing the precision of biometrics by determining whether the biometrics fall within a normal range.

According to the second embodiment, the storage unit 210 may store an image 211 of the object, and biometrics data 212. The storage unit 210 may store the biometrics data 212 including information about the normal range of the biometrics to determine whether measured biometrics fall within the normal range.

According to the second embodiment, the control unit 220 may include a modeling module 221, a measuring module 222, and a calculating module 223.

When biometrics measured by the measuring module 222 do not fall within the normal range, the modeling module 221 may model the object by estimating a case where the biometrics fall within the normal range.

When the biometrics measured by the measuring module 222 do not fall within the normal range, the measuring module 222 measures biometrics of the object, based on the estimated modeling result.

When the biometrics measured by the measuring module 222 do not fall within the normal range and the object is modeled by estimating a case where the biometrics fall within the normal range, the calculating module 223 may calculate an error rate between biometrics measured again by the measuring module 222 and the previously measured biometrics.

The input unit 230 is a unit that generates a user input signal for controlling or operating the terminal apparatus 200, under a user's manipulation, and may be embodied as any of various input units. For example, the input unit 230 may include at least one among a key input unit, a touch input unit, a gesture input unit, a voice input unit, and the like. The key input unit may generate a signal corresponding to a key when the key is manipulated, and may be embodied as a keypad or a keyboard. The touch input unit may recognize a user input by sensing a user's touch on a particular part, and may be embodied as a touch pad, a touch screen, or a touch sensor. The gesture input unit senses a user's predetermined motion, e.g., shaking or moving a terminal, accessing the terminal, or blinking of the user's eyes, as a particular input signal, and may include at least one among a terrestrial magnetism sensor, an acceleration sensor, a camera, an altimeter, a gyro sensor, and a proximity sensor.

The output unit 240 outputs a user interface for providing biometrics and a result of measuring to a screen (not shown) of the terminal apparatus 200. For example, the output unit 240 may be one of a liquid crystal display (LCD), a thin film transistor-LCD (TFT-LCD), light-emitting diodes (LEDs), organic light-emitting diodes (OLEDs), active matrix organic light-emitting diodes (AMOLED), a flexible display, and a three-dimensional (3D) display.

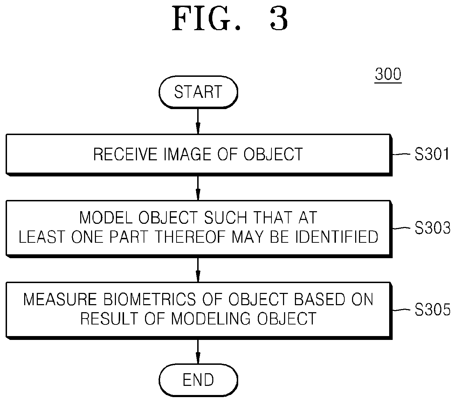

FIG. 3 is a flowchart illustrating a method 300 of measuring biometrics of an object, according to the first embodiment of the present invention.

The method 300 of FIG. 3 may be performed by the terminal apparatus 100 of FIG. 1 according to the first embodiment or the terminal apparatus 200 of FIG. 2 according to the second embodiment.

The method 300 performed by the terminal apparatus 100 or 200 will now be described in detail.

The terminal apparatus 100 or 200 may receive an image of an object from an external storage device or may read an image stored in the storage unit 110 to measure biometrics of the object, according to a request from a user or a control signal (operation S301). The receiving or reading of the image in operation S301 may be performed by the control unit 120 or 220.

Then, the object is modeled based on the image received or read in operation S301 such that at least one part of the object may be identified (operation S303). Operation S303 may be performed by the modeling module 121 or 221.

Then, biometrics of the object may be measured based on a result of modeling the object performed in operation S303 (operation S305).

Operation S305 may be performed by the measuring module 122 or 222.

In this case, the result of modeling the object may be output to a user, and the user may view and modify the result of modeling the object.

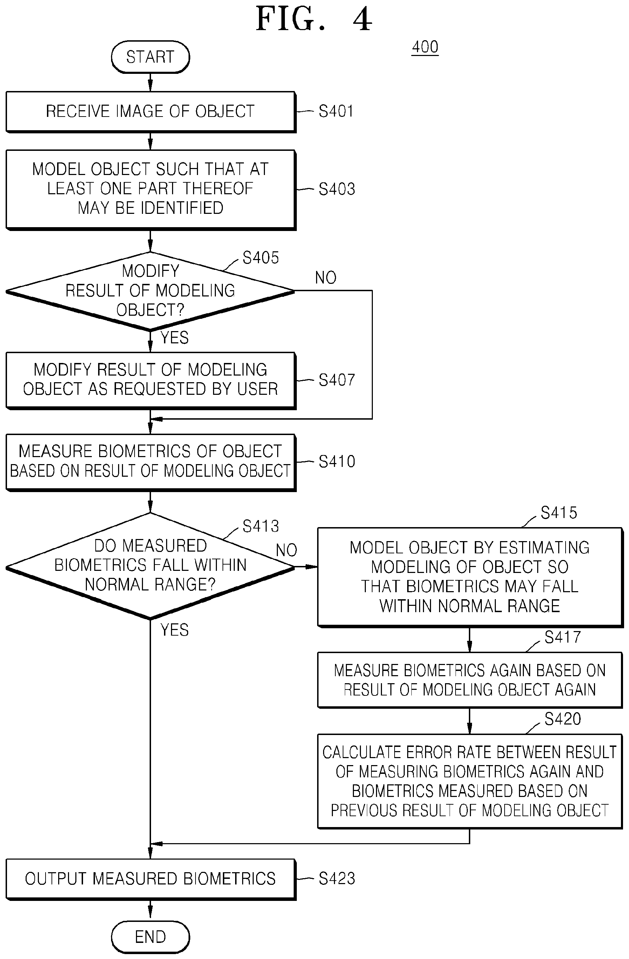

FIG. 4 is a flowchart illustrating a method 400 of measuring biometrics of an object, according to the second embodiment of the present invention.

The method 400 of FIG. 4 may be performed by the terminal apparatus 200 of FIG. 2 according to the second embodiment. Thus, the method 400 performed by the terminal apparatus 200 will now be described in detail.

The terminal apparatus 200 may receive an image of an object from an external storage device or read the image 211 of the object stored in the storage unit 210 so as to measure biometrics of the object, according to a request from a user or a control signal (operation S401). In operation S401, the receiving of the image or the reading of the image 211 may be performed by the control unit 220.

Then, the object may be modeled such that at least one part of the object may be identified, based on the image 211 of the object (operation S403). Operation S403 may be performed by the modeling module 221.

A result of modeling the object may be output to a user via the output unit 240, and the user may view and modify the result of modeling the object. Then, if it is determined that the user checks the result of modeling the object and requests to modify the result of modeling the object via the input unit 230 (operation S405), the result of modeling the object may be modified as requested by the user (operation S407). The request to modify the result of modeling the object in operation S405 may be received via the input unit 230, and operation S407 may be performed by the control unit 220.

If it is determined that the user does not request to modify the result of modeling the object via the input unit 230 (operation S405), biometrics of the object may be measured based on the result of modeling the object (operation S410). Then, whether the measured biometrics fall within a normal range may be determined based on the biometrics data 212 stored in the storage unit 210 of the terminal apparatus 200 (operation S413). If the measured biometrics do not fall within the normal range, the precision of the measured biometrics may be determined to be low.

Otherwise, if the measured biometrics fall within the normal range, the measured biometrics are output (operation S423). If the measured biometrics do not fall within the normal range, the object may be modeled again by estimating a case where the biometrics fall within the normal range (operation S415). Then, biometrics of the object are measured based on the estimated modeling result (operation S417). Then, the biometrics measured again and the biometrics measured based on the previous result of modeling the object are compared to calculate an error rate therebetween (operation S420).

In addition, the terminal apparatus 200 may calculate and output data for diagnosing a state of the object, based on the measured biometrics. If the measured biometrics do not fall within the normal range, the precision of the measured biometrics may be determined to be low. Thus, the data for diagnosing the state of the object may be calculated and output, based on the biometrics measured based on the estimated modeling result.

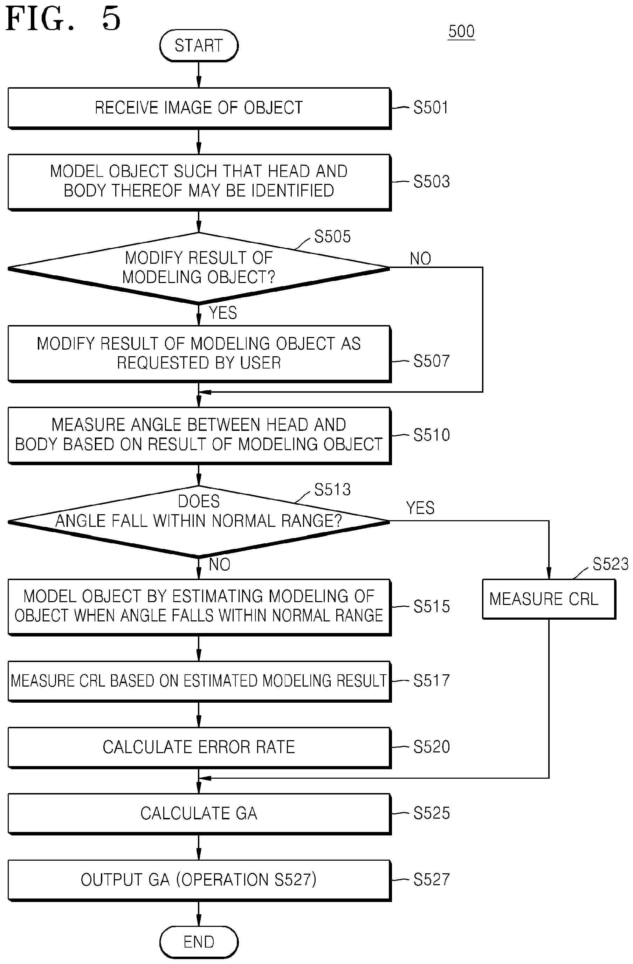

FIG. 5 is a flowchart illustrating a method 500 of measuring a CRL of an object, according to a third embodiment of the present invention.

According to the third embodiment, the object is modeled such that a head and body of the object may be identified to measure the CRL of the object, which is one of biometrics thereof, and modeling of the object may be estimated and performed again to increase the precision of measured biometrics, according to whether an angle between the head and body of the object falls within a normal range.

The terminal apparatus 200 of FIG. 2 may receive an image of an object from an external device or may read the image 211 of the object stored in the storage unit 210 to measure biometrics of the object, according to a request from a user or a control signal (operation S501).

Then, the object may be modeled such that the head and body of the object may be identified, based on the image 211 of the object (operation S503). A result of modeling the object may be output to a user via the output unit 240, and the user may check the result of modeling the object. If the user views the result of modeling the object and requests to modify the result of modeling the object via the input unit 230 (operation S505), the result of modeling the object may be modified as requested by the user (operation S507).

A CRL of the object may be measured based on the result of modeling the object. First, characteristic points of the head and body of the object may be extracted, and a central axis may be set on a figure obtained by modeling the object, based on the extracted characteristic points. Then, an angle between the head and body of the object may be measured based on the central axis (operation S510). Here, the characteristic points may represent a predetermined part of the object, including at least one among the crown of the head, palatine bones, and the end of a nose of the object.

If the angle between the head and body of the object falls within the normal range, then the CRL of the object may be precisely measured. In the case of a fetus, for example, the CRL may be measured to be small when the fetus crouches down to a large extent, and may be measured to be too large when the fetus stretches. Thus, the measured CRL is not appropriate to be used to calculate a gestational age (GA), which is a value for diagnosing a state of the fetus.

Then, whether the angle falls within the normal range may be determined based on information about the normal range of the angle, included in the biometrics data 212 stored in the storage unit 210 of the terminal apparatus 200 (operation S513), thereby enabling the CRL to be precisely measured.

If the angle falls within the normal range, the CRL is measured using the result of modeling the object (operation S523). Otherwise, if the angle does not fall within the normal range, the object is modeled again by estimating a case where the angle falls within the normal range (operation S515). In the case of a fetus, for example, when the angle between the head and body of the object does not fall within the normal range since the fetus crouches down to a large extent, modeling of the object may be estimated and performed again by controlling the figure obtained by modeling the object such that the central axis on the head or body may be moved to a side.

Then, the CRL is measured again based on the estimated modeling result (operation S517). Then, a result of measuring the CRL again and a result of measuring the CRL based on the previous result of modeling the object are compared to calculate an error rate therebetween (operation S520).

Thereafter, a GA, which is a value for diagnosing a state of the fetus, may be calculated based on the CRL (operation S525), and may then be output via the output unit 240 (operation S527).

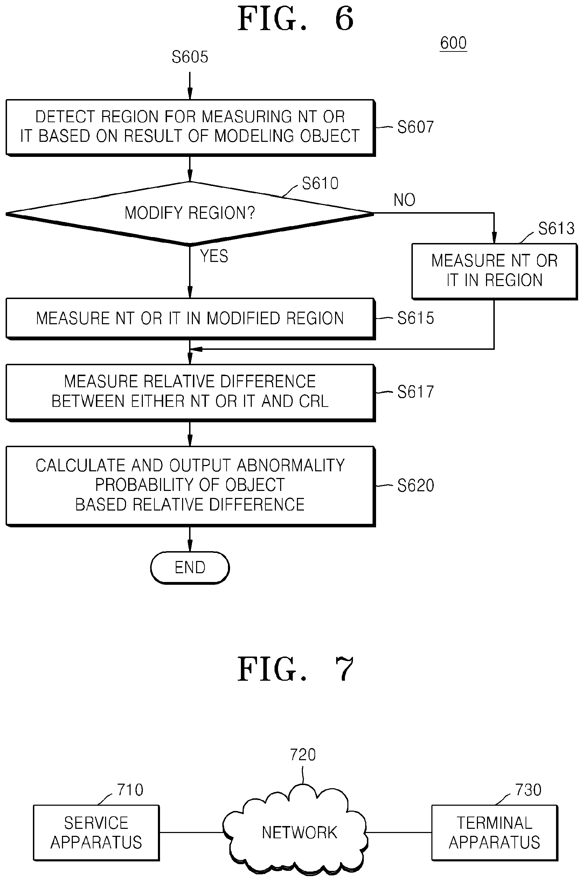

FIG. 6 is a flowchart illustrating a method 600 of measuring an NT or an IT of an object, according to a fourth embodiment of the present invention.

In the fourth embodiment, the IT or the NT of the object may be measured based on a result of modeling the object. The object may be modeled such that a head and body thereof may be identified according to the third embodiment, and the IT or NT of the object may then be measured according to the fourth embodiment. In this case, measuring of the CRL is not obligatory as measuring the IT or NT and may be thus optional. Thus, operation S605 of FIG. 6 may correspond to operation S507 or S505 of FIG. 5.

Referring to FIG. 6, the location of the NT or IT of the object may be estimated based on the result of modeling the object. In the case of a fetus, the NT thereof is the nape and may thus be estimated as a region in which the head and body intersect, and the IT thereof is located in the skull and may thus be estimated to be located in a region in which a central point and a central axis on the head intersect. A region-of-interest (ROI) in which the NT or IT may be measured may be indicated.

A region in which the NT or IT may be measured, i.e., the ROI, may be detected and output based on the result of modeling the object (operation S607). A user may check the output ROI and request to modify the ROI.

Then, if the control unit 220 does not receive the request to modify the ROI from the user (operation S610), the control unit 220 may measure the NT or IT in the ROI (operations S613).

Otherwise, if the control unit 220 receives the request to modify the ROI from the user (operation S610), the control unit 220 may modify the ROI based on the request from the user and may measure the NT or IT in the modified ROI (operation S615).

In this case, the NT and IT are measured as lengths and may thus be displayed in the form of a line, together with the ROI.

When the NT or IT is measured, a relative difference between either the NT or IT and the CRL of the object is calculated (operation S617). Then, an abnormality probability of the object may be calculated and output, based on the relative difference (operation S620). The relative difference may be expressed as NT/CRL or IT/CRL. In this case, the CRL has to be measured to calculate the relative difference between the CRL and the NT or IT. Thus, the CRL may be measured according to the third embodiment, and the IT or NT may be measured according to the fourth embodiment.

FIG. 7 is a block diagram of a system that measures biometrics of an object, according to an embodiment of the present invention.

Referring to FIG. 7, the system may include a service apparatus 710, a network 720, and a terminal apparatus 730.

According to an embodiment of the present invention, biometrics of an object may be measured and a state of the object may be diagnosed according to a computing-based method. Here, the computing-based method means a method in which a device (not shown) that is connected to the terminal apparatus 730 via the network 720 measures the biometrics of the object and diagnoses a state of the object, and only information is input to or output from the terminal apparatus 730. For convenience of explanation, a device that measures the biometrics of the object and diagnoses the state of the object, in response to a request from the terminal apparatus 730 according to a fifth, sixth, seventh, or eighth embodiment of the present invention may be hereinafter referred to as the service apparatus 710.

The service apparatus 710 measures the biometrics of the object based on an image of the object received via the network 720, and provides the terminal apparatus 730 with a result of the measuring and a result of diagnosing the state of the object based on the result of the measuring. More specifically, the object may be modeled such that at least one part thereof may be identified in the image of the object, the biometrics of the object may be measured based on a result of modeling the object, a state of the object may be diagnosed according to a result of the measuring, and a result of the diagnosing may then be provided to the terminal apparatus 730. In this case, the service apparatus 710 may provide a user interface via which the result of modeling the object and the measured biometrics_ may be provided to the terminal apparatus 730 so that a user may view and modify a process of measuring the biometrics of the object.

The service apparatus 710 may operate based on a server-client computing or a cloud computing. In other words, computer resources for measuring the biometrics of the object and diagnosing the state of the object, e.g., at least one among hardware and software, may be provided to the service apparatus 710.

The network 720 provides a path for exchanging data between the service apparatus 710 and the terminal apparatus 730. The network 720 is an internet protocol (IP) network via which a service for receiving/transmitting a large amount of data and a data service are provided in a seamless manner by using an IP. The network 720 may be an all-IP network that is an IP network structure obtained by integrating different networks based on an IP. Also, the network 720 may include at least one network from among a 3-generation (G) mobile network including a wired network, a wireless broadcasting (Wibro) network, a wideband code division multiple access (WCDMA) network, a 3.5-G mobile network including a high-speed downlink packet access (HSDPA) network and a long-term evolution (LTE) network, a 4-G mobile network including LTE advanced, and a wireless local area network (LAN) including a satellite network and a Wi-Fi network.

According to the fifth to eighth embodiments, the terminal apparatus 720 only performs an operation of outputting the result of measuring the biometrics of the object and the result of diagnosing the state of the object, performed by the service apparatus 720. Accordingly, the present invention will be hereinafter described focusing on the service apparatus 710.

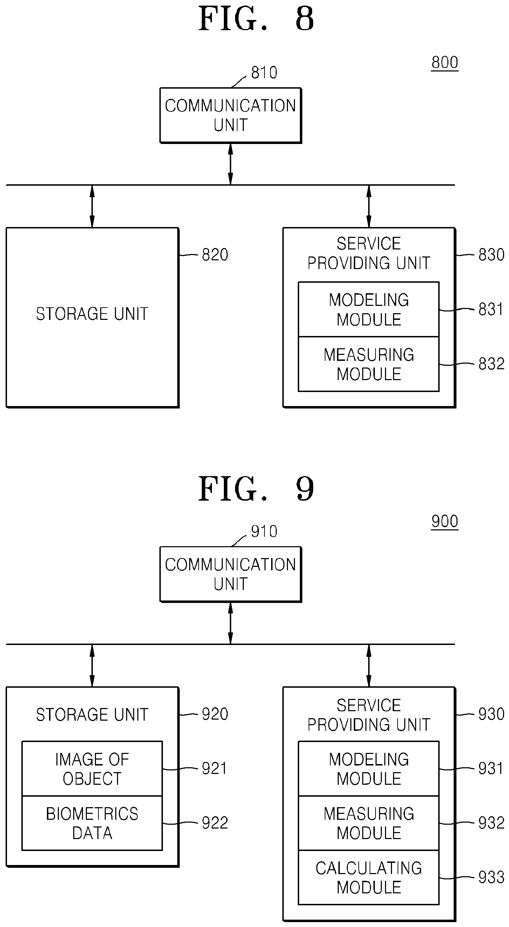

FIG. 8 is a block diagram of a service apparatus 800 included in a system that measures biometrics of an object, according to a fifth embodiment of the present invention. The service apparatus 800 of FIG. 8 may correspond to the service apparatus 710 of FIG. 7 or a service apparatus 900 of FIG. 9.

Referring to FIG. 8, the service apparatus 800 may include a communication unit 810, a storage unit 820, and a service providing unit 830.

The communication unit 810 exchanges data with the terminal apparatus 730 of FIG. 7 via the network 720 of FIG. 7.

The storage unit 820 stores data and a program for operating the service apparatus 800. In the fifth embodiment, the storage unit 820 may store an image of an object. In this case, the image of the object may include an internal or external image of the object for measuring biometrics of the object, e.g., an ultrasound image, an MRI image, a CT image, or an X-ray image of the object. The storage unit 820 may be any of various storage media, such as a RAM, a ROM, an HDD, a flash memory, a CD-ROM, and a DVD.

The service providing unit 830 may control such that the image of the object may be received from an external device (not shown) or the storage unit 820, the object may be modeled to identify at least one part thereof, based on the image of the object, biometrics of the object may be measured based on a result of modeling the object, and the measured biometrics may be then output to an external display unit (not shown) or an output unit (not shown) in the terminal apparatus 100 of FIG. 1.

According to the fifth embodiment, the service providing unit 830 may include a modeling module 831 and a measuring module 832.

The modeling module 831 models the object such that respective parts of the object may be identified, based on the image of the object. The object may be modeled in an oval shape including a circular shape, but is not limited thereto. When the object is a fetus, parts of the fetus may be largely divided into a head and a body, and the head and body of the fetus may be modeled in a circular or oval shape and then be provided to an output unit (not shown).

When the modeling module 831 models the object to identify the respective parts thereof, the measuring module 832 measures the biometrics of the object based on a result of modeling the object. If the object is a fetus, a central axis may be set on the circular or oval shape by using characteristic points of the head and body thereof, and a CRL, NT, and IT of the fetus, which are biometrics thereof, may be measured based on the set central axis.

FIG. 9 is a block diagram of a service apparatus 900 included in a system that measures biometrics of an object, according to a sixth embodiment of the present invention.

Referring to FIG. 9, the service apparatus 900 according to the sixth embodiment may include a communication unit 910, a storage unit 920, and a service providing unit 930. The communication unit 910, the storage unit 920, and the service providing unit 930 correspond to the communication unit 810, the storage unit 820, and the service providing unit 830 of FIG. 8, respectively., and thus, repeated descriptions thereof are not provided again.

According to the sixth embodiment, the service apparatus 900 may provide the terminal apparatus 730 of FIG. 7 with a method of measuring biometrics of an object, which is capable of increasing the precision of biometrics by determining whether the biometrics fall within a normal range.

According to the sixth embodiment, the storage unit 920 may store an image 921 and biometrics data 922 of an object. The storage unit 920 further stores the biometrics data 922 including information about a normal range of at least one biometric, thereby enabling to determine whether measured biometrics fall within the normal range.

According to the sixth embodiment, the service providing unit 930 may include a modeling module 931, a measuring module 932, and a calculating module 933.

If biometrics measured by the measuring module 932 do not fall within the normal range, the modeling module 931 models the object again such that biometrics of the object may fall within the normal range.

If the measured biometrics do not fall within the normal range, the measuring module 932 measures biometrics of the object again, based on a result of modeling the object again, performed by the modeling module 931.

If the biometrics measured by the measuring module 932 do not fall within the normal range and the object is modeled again by estimating a case where biometrics of the object fall within the normal range, the calculating module 933 calculates an error rate between the biometrics measured again by the measuring module 932 and the previously measured biometrics and provides the error rate to a user so that the user may determine the precision of the previously measured biometrics.

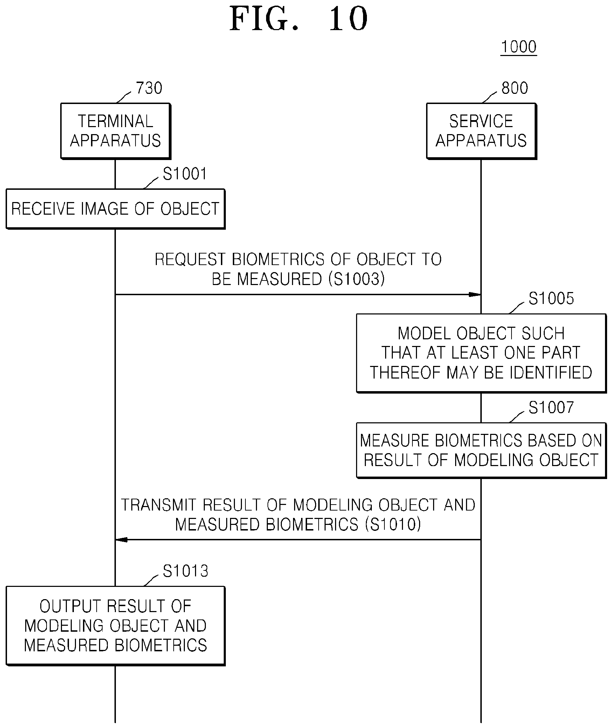

FIG. 10 is a flowchart illustrating a method 1000 of measuring biometrics of an object, according to the fifth embodiment of the present invention.

Referring to FIG. 10, a terminal apparatus 730 may receive an image of an object from an external device or may read an image stored in a storage unit therein so as to measure biometrics of the object, according to a request from a user or a control signal (operations S1001). Then, the terminal apparatus 730 may transmit the image of the object to a service apparatus 800 so as to request to measure biometrics of the object (operation S1003). The image of the object may be stored in the service apparatus 800. In this case, the terminal apparatus 730 may request the service apparatus 800 to measure the biometrics of the object stored in the service apparatus 800 and provide the terminal apparatus 730 with a result of the measuring.

Then, the service apparatus 800 may model the object such that at least one part of the object may be identified, based on the image of the object (operation S1005). Then, the service apparatus 800 may measure biometrics of the object based on a result of modeling the object (operation S1007).

Then, the result of modeling the object and the measured biometrics may be transmitted to the terminal apparatus 730 (operation S1010). Then, the result of modeling the object and the measured biometrics may be output to the user via the terminal apparatus 730 (operation S1013). Thus, the user may view and modify the result of modeling the object and the measured biometrics.

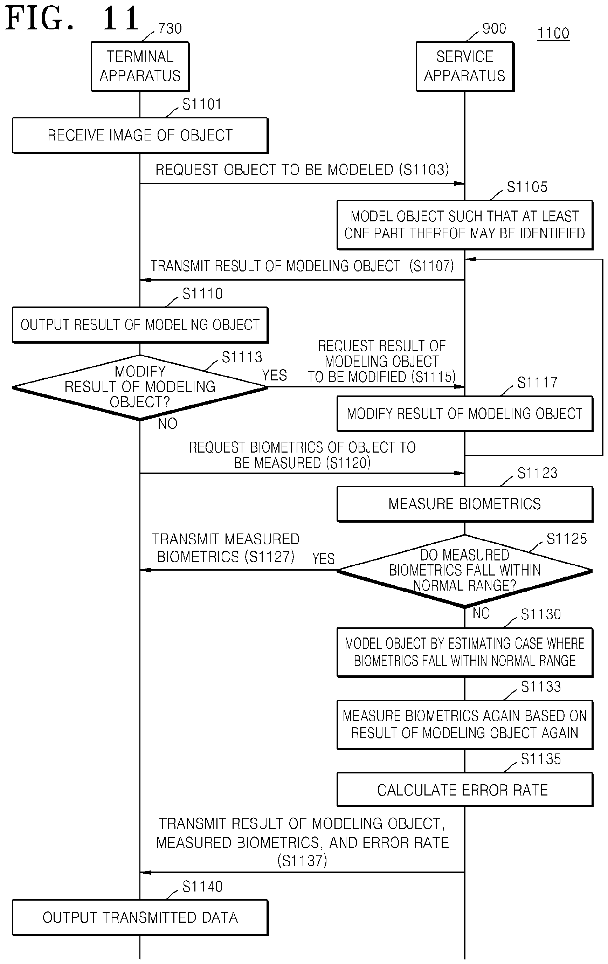

FIG. 11 is a flowchart illustrating a method 1100 of measuring biometrics of an object, according to the sixth embodiment of the present invention.

Referring to FIG. 11, a terminal apparatus 730 may receive an image of an object from an external device or may read an image stored in a storage unit therein so as to measure biometrics of the object, according to a request from a user or a control signal (operation S1101). Then, the terminal apparatus 730 may transmit the image to a service apparatus 900 to request the service apparatus 900 to model the object in order to measure biometrics of the object (operation S1103). The image of the object may be stored in the service apparatus 900. In this case, the terminal apparatus 730 may request the service apparatus 900 to model the image of the object stored in the service apparatus 900.

Then, the object may be modeled such that at least one part of the object may be identified, based on the image (operation S1105). Then, a result of modeling the object may be transmitted to the terminal apparatus 730 (operation S1107). Then, the result of modeling the object may be output to the user via the output unit (not shown) in the terminal apparatus 730 (operation S1110). Then, when the user views the result of modeling the object and requests the service apparatus 900 to modify the result of modeling the object, via an input unit (not shown) of the terminal apparatus 730 (operations S1113 and S1115), the result of modeling the object may be modified as requested by the user (operation S1117).

When a request to modify the result of modeling the object is not received from the user, the service apparatus 900 may be requested to measure biometrics of the object (operation S1120). Then, the service apparatus 900 may measure biometrics of the object, based on the result of modeling the object (operation S1123).

Then, whether the measured biometrics fall within a normal range may be determined based on the biometrics data 922 stored in the storage unit 920 of the service apparatus 900 (operation S1125). If the measured biometrics do not fall within the normal range, the precision of the measured biometrics may be determined to be low.

Otherwise, if the measured biometrics fall within the normal range, the measured biometrics may be transmitted to the terminal apparatus 730 (operation S1127). Then, the measured biometrics may be output to the user via the output unit (not shown) in the terminal apparatus 730 (operation S1140). If the measured biometrics do not fall within the normal range, the object is modeled again by estimating a case where biometrics of the object fall within the normal range (operation S1130). Then, the biometrics of the object are measured again based on a result of modeling the object again (operation S1133). Then, the measured biometrics and the biometrics measured based on the previous result of modeling the object may be compared to calculate an error rate therebetween (operation S1135).

In addition, the service apparatus 900 may calculate data for diagnosing a state of the object from the measured biometrics and provide the data to the terminal apparatus 730. However, if the measured biometrics do not fall within the normal range, the precision of the measured biometrics may be determined to be low. Thus, the data for diagnosing the state of the object may be calculated from the biometrics measured based on the estimated modeling result, and then be provided to the terminal apparatus 730.

Thereafter, data related to the biometrics of the object, including the result of modeling the object, the measured biometrics, the error rate, and the like, may be transmitted to the terminal apparatus 730 (operation S1137). Then, the data may be controlled to be output by the terminal apparatus 730 (operation S1140).

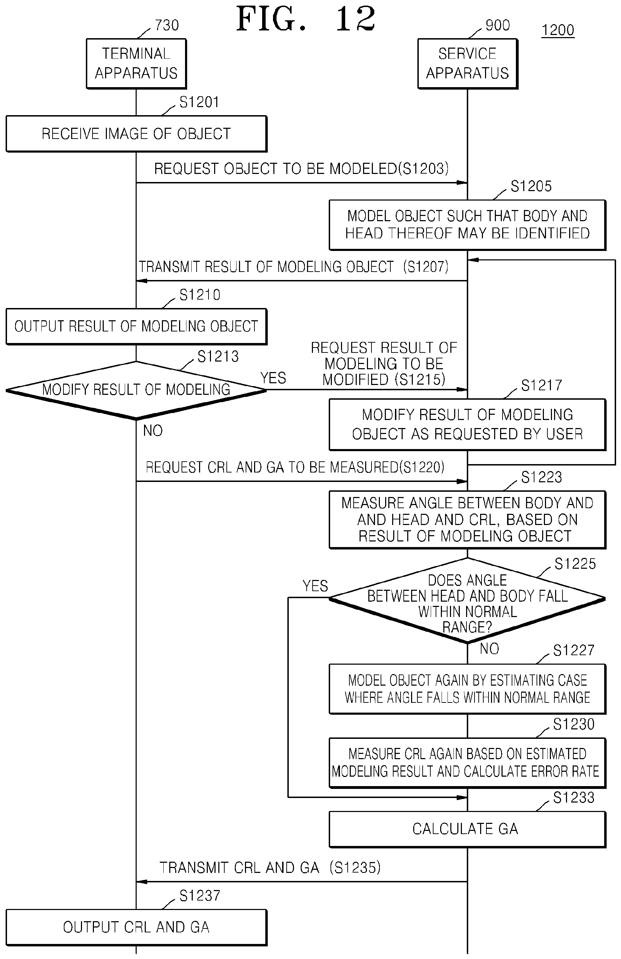

FIG. 12 is a flowchart illustrating a method 1200 of measuring a CRL of an object, according to a seventh embodiment of the present invention.

According to the seventh embodiment, in order to measure a CRL, which is one of biometrics of an object, the object may be modeled such that a body and head of the object may be identified, and may be modeled again according to whether an angle between the body and head falls within a normal range.

Referring to FIG. 12, a terminal apparatus 730 may receive an image of an object from an external device or may read an image from a storage unit therein to measure biometrics of the object, according to a request from a user or a control signal (operation S1201). Then, the terminal apparatus 730 may transmit the image to the service apparatus 900 to request the service apparatus 900 to model the object so as to measure biometrics of the object (operation S1203). The image of the object may be stored in the service apparatus 900. In this case, the terminal apparatus 730 may request the service apparatus 900 to model the image of the object stored in the service apparatus 900 and provide a result of modeling the object.

Then, the object may be modeled such that a body and head thereof may be identified, based on the image of the object (operation S1205). Then, a result of modeling the object may be transmitted to the terminal apparatus 730 (operation S1207). Then, the result of modeling the object may be output to a user via the output unit of the terminal apparatus 730 (operation S1210). When the user views the result of modeling the object and requests the service apparatus 900 to modify the result of modeling the object, via the input unit of the terminal apparatus 730 (operations S1213 and S1215), the result of modeling the object may be modified as requested by the user (operation S1217).

If there is no request ,from the user to modify the result of modeling the object and the terminal apparatus 730 requests the service apparatus 900 to provide biometrics of the object (operation S1220), then the service apparatus 900 may measure biometrics of the object, based on the result of modeling the object (operation S1223).

For example, a CRL of the object may be measured based on the result of modeling the object. Then, a GA may be calculated from the CRL. First, characteristic points on the head and body of the object may be extracted, a central axis may be set on a figure obtained by modeling the object, based on the extracted characteristics points, and then, biometrics of the object may be measured. In operation S1223, an angle between the body and head of the object and the CRL of the object may be measured based on the central axis.

When the angle between the head and body of the object falls within the normal range, the CRL of the object may be precisely measured. In the case of a fetus, the CRL may be measured to be small when the fetus crouches down to a large extent and may be measured to be too large when the fetus stretches. Thus, the measured CRL is not appropriate for calculating a GA, which is a value for diagnosing a state of the fetus.

Thus, whether the angle between the head and body of the object falls within the normal range may be determined based on information about the normal range of this angle, included in the biometrics data 922 stored in the storage unit 920 of the service apparatus 900 (operation S1225), thereby enabling the CRL to be precisely measured.

If the angle between the head and body of the object does not fall within the normal range, the object is modeled again by estimating a case where the angle falls within the normal range (operation S1227). In the case of a fetus, if the angle between the head and body of the object does not fall within the normal range since the fetus crouches down to a large extent, modeling of the object may be estimated and performed again by controlling a figure obtained by modeling the object such that the central axis on the head or body may be moved to a side and the angle may thus fall within the normal range.

Then, a CRL of the object may be measured again based on a result of modeling the object again, and the measured CRL and the CRL measured based on the previous result of modeling the object may be compared to calculate an error rate therebetween (operation S1230).

In addition, a GA for diagnosing a state of a fetus may be calculated from the CRL (operation S1233). Then, the CRL and GA may be transmitted to the terminal apparatus 730 (operation S1235) and may be output via the output unit of the terminal apparatus 730 (operation S1237.

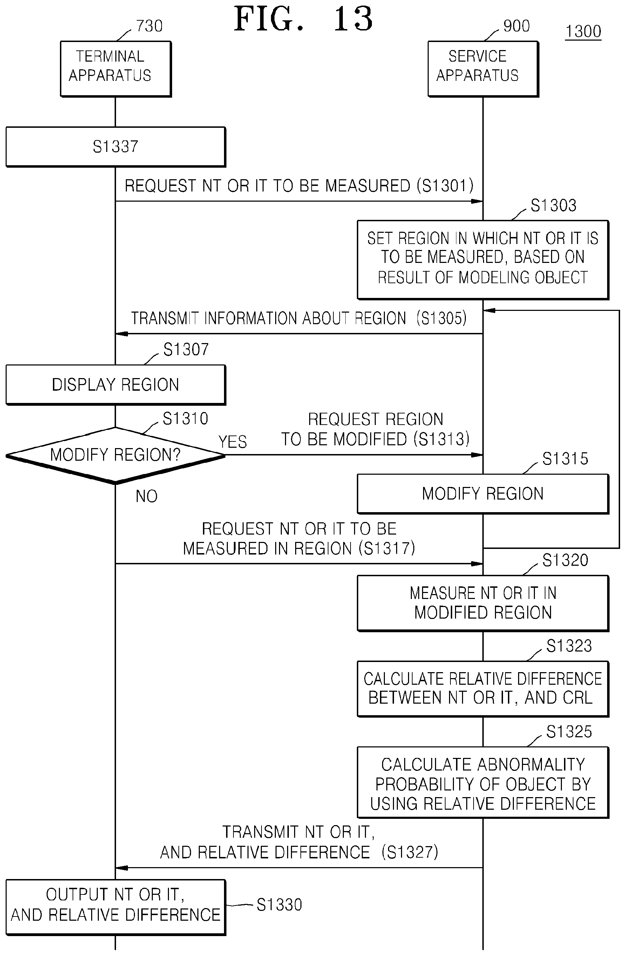

FIG. 13 is a flowchart illustrating a method 1300 of measuring an NT or an IT of an object, according to an eighth embodiment of the present invention.

According to the eighth embodiment, an IT or NT of an object may be measured based on a result of modeling the object. Thus, the object may be modeled such that the head and body thereof may be identified according to the seventh embodiment, and the IT or NT of the object may then be measured based on a result of modeling the object. In this case, measuring a CRL of the object is not obligatory as measuring the IT or NT and may be optionally performed. Accordingly, operation S1337 of FIG. 13 may correspond to operation S1237 of FIG. 12.

Referring to FIG. 13, locations of the NT or IT of the object may be estimated based on a result of modeling the object. In the case of a fetus, the NT thereof is the nape and may thus be estimated as a region in which the head and body intersect, and the IT thereof is located in the skull and may thus be estimated to be located in a region in which a central point and a central axis on the head intersect. An ROI in which the NT or IT may be measured may be indicted.

First, a terminal apparatus 730 requests a service apparatus 900 to measure an NT or IT of an object and provide a result of the measuring, according to a request from a user of the terminal apparatus 730 or a control signal (operation S1301). Then, the service apparatus 900 sets a region in which the NT or IT is to be measured, i.e., an ROI, based on a result of modeling the object (operation S1303). When the set ROI should be set to be verified by a user, information about the set ROI may be transmitted to the terminal apparatus 730 (operation S1305). Then, the ROI may be displayed on the terminal apparatus 730 (operation S1307)

When the user views the displayed ROI and requests to modify the ROI, (operations S1310 and S1313), the ROI is modified as requested by the user (operation S1315). Then, the NT or IT may be requested to be measured in the modified ROI (operation S1317), and then be measured in the modified ROI (operation S1320).

In this case, the NT and IT are measured as lengths and may thus be displayed in the form of a line, together with the ROI.

After the NT or IT is measured, a relative difference between either the NT or IT and the CRL may be calculated (operation S1323). Then, an abnormality probability of the object may be calculated using the relative difference and then be provided to the terminal apparatus 730 (operation S1325). In this case, the relative difference may be expressed as NT/CRL or IT/CRL. The CRL has to be measured to calculate the relative difference between the CRL and the NT or IT. The CRL may be measured according to the third embodiment, and the IT or NT may then be measured according to the fourth embodiment.

Then, the measured NT or IT, and the relative difference between the NT or IT and the CRL may be transmitted to the terminal apparatus 730 (operation S1327), and may then be output via the output unit of the terminal apparatus 730 (operation S1330).

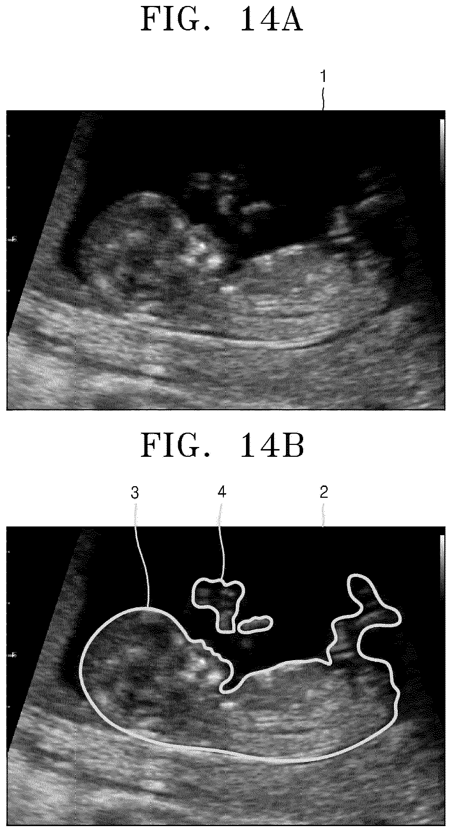

FIGS. 14A and 14B illustrate examples of an ultrasound image of an object transmitted to a terminal apparatus or a service apparatus according to an embodiment of the present invention.

Specifically, FIG. 14A illustrates an example of an ultrasound image 1 of a fetus, received, for example, by the control unit 120 or 220 of FIG. 1 or 2 or the service providing unit 830 or 930 of FIG. 8 or 9. The ultrasound image 1 includes a cross-section of the fetus, based on which biometrics of the fetus may be measured.

Referring to FIG. 14B, biometrics of the fetus may be measured by extracting parts 3 and 4 of an ultrasound image 2 of the fetus.

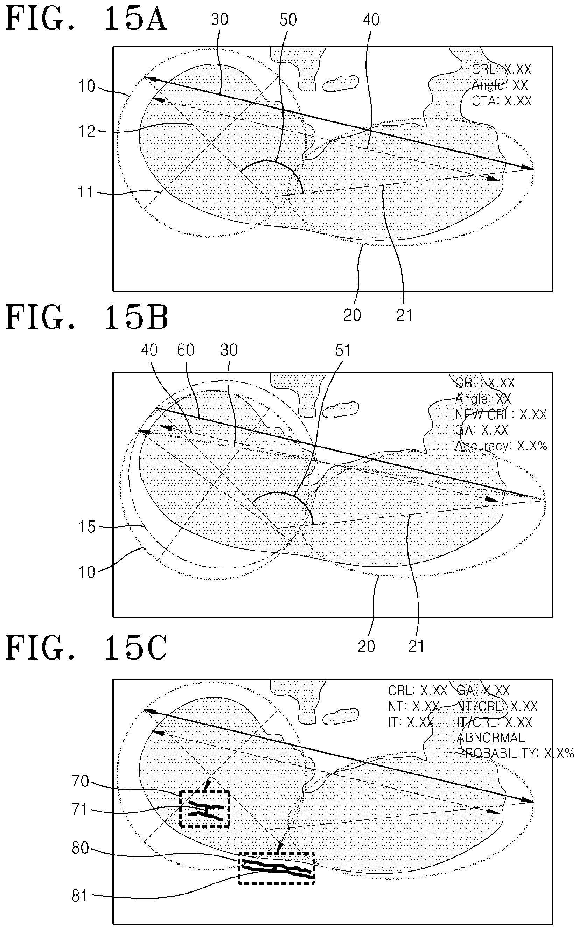

An image of an object illustrated in FIG. 15 may be the same as the ultrasound image of FIG. 14A or 14B or may be obtained by extracting a part of the ultrasound image 1 or 2 of FIG. 14A or 14B.

FIGS. 15A to 15C illustrate images each showing a result of modeling an object and a result of measuring a CRL, IT, and NT of the object, according to embodiments of the present invention.

FIG. 15A illustrates an image showing, when an object is a fetus, a result of modeling the fetus and a result of measuring a CRL of the fetus.

Referring to FIG. 15A, the fetus may be modeled such that the head and body thereof are identified in a circular shape 10 and an oval shape 20, respectively, characteristic points on the head and body may be extracted, and central axes 11, 12, and 21 are then set and indicated based on the characteristic points.

A CRL 30 may be automatically displayed and measured based on the central axes 11, 12, and 21. A user may select a desired part of the fetus to be measured and may manually measure a CRL 40.

An angle 50 between the head and body may also be measured with respect to the central axes 11, 12, and 21. Then, whether the angle 50 falls within a normal range may be determined.

FIG. 15B illustrates an image showing, when an object is a fetus, a result of modeling the fetus and a result of measuring a CRL of the fetus when an angle between the head and body does not fall within a normal range.

If the angle between the head and body of the object does not fall within the normal range, the object is modeled by estimating a case where the angle between the head and body falls within the normal range. Referring to FIG. 15B, if the angle falls beyond the normal range, a result of modeling the head is moved toward a result of modeling the body (as indicated with a line 15) in order to adjust the angle between the head and body to fall within the normal range.

FIG. 15C illustrates an image showing, when an object is a fetus, a result of modeling the fetus and a result of measuring an IT and NT of the fetus.

The NT and IT may be measured by setting regions in which central axes on parts of the object that is modeled contact a circular shape and an oval shape with which the head and body of the fetus are modeled, as ROIs. Referring to FIG. 15C, a central point on the head and a region in which the head and body contact each other may be set as an ROI 70 of the NT and an ROI 80 of the IT, respectively, and then be displayed to be expanded. Also, parts of the ROIs 70 and 80 in which the NT and IT are to be measured may be displayed as lines 71 and 81. Then, a user may view displayed information and may directly modify the ROIs 70 and 80 or the parts of the ROIs 70 and 80 in which the NT and IT are to be measured.

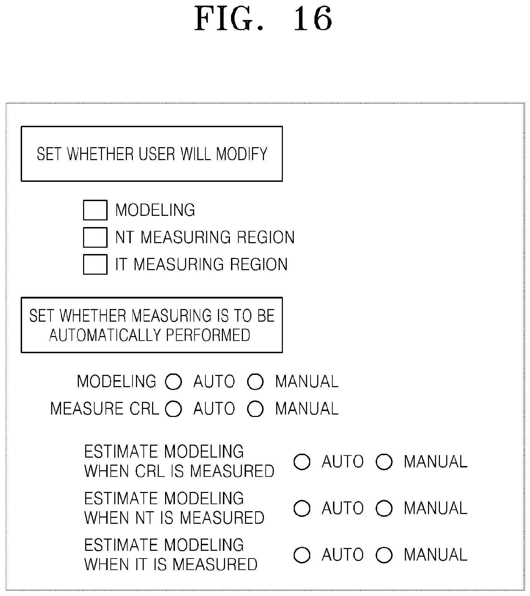

FIG. 16 illustrates a screen image on which whether an object is to be modeled, whether biometrics of the object are to be automatically measured, and whether the measured biometrics are to be verified by a user after the measurement of the biometrics may be set.

Referring to FIG. 16, a user interface via which the biometrics of the object may be set to be measured after a user verifies a result of modeling the object, an NT measuring region, and an IT measuring region may be provided. If the biometrics are set to be measured after the user verifies the result of modeling the object, the NT measuring region, and the IT measuring region, then the result of modeling the object or the NT measuring region and the IT measuring unit may be automatically set and displayed, whether these displayed items are to be modified may be determined according to the user's verification thereof, and then the biometrics of the object may be measured.

Also, after modeling of the object, the result of modeling the object, the NT measuring region, and the IT measuring region are determined, the user interface of FIG. 16 may be provided so that a user may determine whether biometrics of the object, such as a CRL, an NT, and an IT, are to be automatically or manually measured. In this case, if the biometrics of the object are determined to be automatically measured, the biometrics are measured by setting measuring regions based on a result of modeling the object. If the biometrics of the object are determined to be manually measured, the biometrics are measured by manually modeling the object or setting measuring regions by the user. For example, when the biometrics are measured by measuring lengths of parts of the object, it is possible to set such that a user may make dots on an image of the object to measure the lengths.

The present invention can be embodied as code that may be read by a computer (including various devices capable of processing information), in a computer-readable recording medium. Here, the computer-readable recording medium may be any recording apparatus capable of storing data that is read by a computer system, e.g., a read-only memory (ROM), a random access memory (RAM), a compact disc (CD)-ROM, a magnetic tape, a floppy disk, an optical data storage device, and so on.

Although a few embodiments of the present invention have been shown and described focusing on novel characteristics of the present invention, it would be appreciated by those of ordinary skill in the art that changes may be made in these exemplary embodiments without departing from the invention, the scope of which is defined in the claims.

## Claims

1. A method of measuring biometrics of an object, the method comprising:
receiving an image of the object (S301);
modeling the object such that at least one part of the object is identified (S303); and
measuring biometrics of the object, based on a result of modeling the object (S305);
wherein the object is a fetus; wherein:
the biometrics is a crown-rump length (CRL) of the fetus and the modeling of the object comprises modeling the object such that a body (20) and head (10) of the object are identified (S503), and after the modeling of the object, the method further comprising:
detecting at least one characteristic point on the head of the fetus;
setting a central axis (21) by using the at least one characteristic point, and displaying the central axis;
measuring an angle (51) between the body and head of the fetus with respect to the central axis (S510);
determining whether the angle falls within a normal range (S513); and
measuring a crown-rump length (CRL) of the fetus,
wherein the measuring of the CRL comprises:
if the angle falls within the normal range, measuring the CRL of the fetus based on the result of modeling the fetus (S523);
if the angle does not fall within the normal range, estimating a model of the fetus corresponding to a case where the angle falls within the normal range (S515), and measuring the CRL of the fetus, based on the estimated modeling result (S517); and
displaying the estimated modeling result and the measured CRL.

2. The method of claim 1, wherein the measuring of the biometrics of the object comprises:
detecting a region-of-interest, ROI, (S607)for measuring the biometrics, based on the result of modeling the object; and
measuring the biometrics in the ROI (S615).

3. The method of claim 2, wherein the detecting of the ROI comprises:
displaying the detected ROI to be differentiated from the other parts of the object;
displaying a region for measuring the biometrics, in the ROI; and
modifying the ROI or the region for measuring the biometrics (S610), according to a user input signal.

4. The method of claim 1, wherein the measuring of the biometrics comprises:
measuring a crown-rump length (CRL) of the fetus (S1223);
measuring at least one among a nuchal translucency (NT) and an intracranial translucency (IT) of the fetus (S1320);
calculating a relative difference between the CRL and either the NT or the IT (S1323); and
displaying the measured CRL, NT, and IT (S1330).

5. A terminal apparatus for measuring biometrics of a fetus, the terminal apparatus comprising:
a storage unit for storing an image of the fetus (110); and
a control unit (120) comprising:
a modeling module for modeling the fetus such that at least one part of the fetus is identified in the image of the object; and
a measuring module for measuring biometrics of the fetus, based on a result of modeling the fetus; wherein:
the modeling module is arranged to model the fetus such that a body and head of the fetus are identified and to detect at least one characteristic point on the head of the fetus, and
to set a central axis by using the at least one characteristic point,
the storage unit is adapted to store biometrics data including information about a normal range of at least one biometric,
the measuring module is arranged to measure an angle between the body and head of the fetus, to determine whether the angle falls within a normal range, and to measure a crown-rump length (CRL) of the fetus, and
the terminal apparatus further comprises an output unit for outputting a result of estimating a result of modeling the fetus when the angle falls within a normal range and a result of measuring the CRL of the fetus;
if the angle falls within the normal range, the measuring module measures the CRL of the fetus based on the result of modeling the fetus, and
if the angle does not fall within the normal range, the measuring module estimates a model of the fetus corresponding to a case where the angle falls within the normal range, and measures the CRL of the fetus based on the estimated modeling result.

6. The terminal apparatus of claim 5, wherein the measuring module is arranged to detect a region-of-interest (ROI) for measuring the biometrics, based on the result of modeling the object, and to measure the biometrics in the ROI, and
the terminal apparatus further comprises an output unit for outputting the detected ROI to be differentiated from the other parts of the object, and outputting a region for measuring the biometrics in the ROI.

7. The terminal apparatus of claim 5, wherein the control unit further comprises a calculating module for calculating an error rate between a result of measuring the biometrics again and the biometrics measured using the result of modeling the object.

8. The terminal apparatus of claim 5, wherein the measuring module is adapted to measure the CRL of the fetus, and measures at least one among a nuchal translucency (NT) and an intracranial translucency (IT) of the fetus, and
the control unit further comprises a calculating module for calculating a relative difference between the CRL and either the NT or IT.

9. The terminal apparatus of claim 5, wherein the control unit is adapted to provide a user interface via which whether at least one among modeling the fetus and estimating a result of modeling the fetus is to be performed automatically or manually is set according to a user input signal.

## Patentansprüche

1. Verfahren zur Messung der Biometrik eines Objekts, wobei das Verfahren aufweist:
Empfangen eines Bildes des Objekts (S301);
Modellieren des Objekts, so dass mindestens ein Teil des Objekts identifiziert wird (S303); und
Messen der Biometrik des Objekts, basierend auf einem Ergebnis der Modellierung des Objekts (S305);
wobei das Objekt ein Fötus ist;
wobei:
die Biometrik eine Scheitel-Steiß-Länge ("crown-rump-length") (CRL) des Fötus ist und das Modellieren des Objekts ein derartiges Modellieren des Objekts, dass ein Körper (20) und ein Kopf (10) des Objekts identifiziert werden (S503), aufweist, und nach dem Modellieren des Objekts das Verfahren ferner aufweist:
Detektieren mindestens eines charakteristischen Punktes auf dem Kopf des Fötus;
Einstellen einer Mittelachse (21) unter Verwendung des mindestens einen charakteristischen Punktes, und Anzeigen der Mittelachse;
Messen eines Winkels (51) zwischen dem Körper und dem Kopf des Fötus in Bezug auf die Mittelachse (S510);
Bestimmen, ob der Winkel in einen normalen Bereich fällt (S513); und
Messen einer Scheitel-Steiß-Länge (CRL) des Fötus,
wobei das Messen der CRL aufweist:
wenn der Winkel in den normalen Bereich fällt, Messen der CRL des Fötus basierend auf dem Ergebnis der Modellierung des Fötus (S523);
wenn der Winkel nicht in den normalen Bereich fällt, Schätzen eines Modells des Fötus, das einem Fall entspricht, in dem der Winkel in den normalen Bereich fällt (S515), und Messen der CRL des Fötus basierend auf dem geschätzten Modellierungsergebnis (S517); und
Anzeigen des geschätzten Modellierungsergebnisses und der gemessenen CRL.

2. Verfahren nach Anspruch 1, wobei das Messen der Biometrik des Objekts aufweist:
Detektieren eines Bereichs von Interesse ("region of interest"), ROI, (S607) zur Messung der Biometrik, basierend auf dem Ergebnis der Modellierung des Objekts; und
Messen der Biometrik in dem ROI (S615).

3. Verfahren nach Anspruch 2, wobei das Detektieren des ROI aufweist:
Anzeigen des detektierten ROI, der von den anderen Teilen des Objekts unterschieden werden soll;
Anzeigen eines Bereichs zur Messung der Biometrik in dem ROI; und
Modifizieren des ROI oder des Bereichs zur Messung der Biometrik (S610) gemäß einem Benutzereingabesignal.

4. Verfahren nach Anspruch 1, wobei das Messen der Biometrik aufweist:
Messen einer Scheitel-Steiß-Länge (CRL) des Fötus (S1223);
Messen mindestens eines von einer Nackentransparenz (NT) und einer intrakraniellen Transluzenz (IT) des Fötus (S1320);
Berechnen eines relativen Unterschieds zwischen der CRL und entweder der NT oder der IT (S1323); und
Anzeigen der gemessenen CRL, NT und IT (S1330).

5. Terminalvorrichtung zur Messung der Biometrik eines Fötus, wobei die Terminalvorrichtung aufweist:
eine Speichereinheit zum Speichern eines Bildes des Fötus (110); und
eine Steuerungseinheit (120), aufweisend:
ein Modellierungsmodul zur Modellierung des Fötus, so dass mindestens ein Teil des Fötus in dem Bild des Objekts identifiziert wird; und
ein Messmodul zur Messung der Biometrik des Fötus, basierend auf einem Ergebnis der Modellierung des Fötus;
wobei:
das Modellierungsmodul ausgelegt ist, den Fötus so zu modellieren, dass ein Körper und ein Kopf des Fötus identifiziert werden, und mindestens einen charakteristischen Punkt auf dem Kopf des Fötus zu detektieren und eine Mittelachse unter Verwendung des mindestens einen charakteristischen Punktes einzustellen,
die Speichereinheit angepasst ist, biometrische Daten zu speichern, die Informationen über einen normalen Bereich von mindestens einer Biometrik aufweisen,
das Messmodul ausgelegt ist, einen Winkel zwischen dem Körper und dem Kopf des Fötus zu messen, um zu bestimmen, ob der Winkel in einen normalen Bereich fällt, und eine Scheitel-Steiß-Länge (CRL) des Fötus zu messen, und
die Terminalvorrichtung ferner eine Ausgabeeinheit zum Ausgeben eines Ergebnisses einer Schätzung eines Ergebnisses einer Modellierung des Fötus, wenn der Winkel in einen normalen Bereich fällt, und eines Ergebnisses der Messung der CRL des Fötus aufweist;
wenn der Winkel in den normalen Bereich fällt, das Messmodul die CRL des Fötus basierend auf dem Ergebnis der Modellierung des Fötus misst, und wenn der Winkel nicht in den normalen Bereich fällt, das Messmodul ein Modell des Fötus, das einem Fall entspricht, in dem der Winkel in den normalen Bereich fällt, schätzt, und die CRL des Fötus basierend auf dem geschätzten Modellierungsergebnis misst.

6. Terminalvorrichtung nach Anspruch 5, wobei das Messmodul ausgelegt ist, einen Bereich von Interesse (ROI) zur Messung der Biometrik basierend auf dem Ergebnis der Modellierung des Objekts zu detektieren und die Biometrik in dem ROI zu messen, und
die Terminalvorrichtung ferner eine Ausgabeeinheit zum Ausgeben des detektierten ROI, der von den anderen Teilen des Objekts unterschieden werden soll, und Ausgeben eines Bereichs zur Messung der Biometrik in dem ROI aufweist.

7. Terminalvorrichtung nach Anspruch 5, wobei die Steuerungseinheit ferner ein Berechnungsmodul zur Berechnung einer Fehlerrate zwischen einem Ergebnis der erneuten Messung der Biometrik und der mit dem Ergebnis der Modellierung des Objekts gemessenen Biometrik aufweist.

8. Terminalvorrichtung nach Anspruch 5, wobei das Messmodul angepasst ist, die CRL des Fötus zu messen, und mindestens eines von einer Nackentransparenz (NT) und einer intrakraniellen Transluzenz (IT) des Fötus misst, und
die Steuerungseinheit ferner ein Berechnungsmodul zum Berechnen eines relativen Unterschieds zwischen der CRL und entweder der NT oder der IT aufweist.

9. Terminalvorrichtung nach Anspruch 5, wobei die Steuerungseinheit angepasst ist, eine Benutzerschnittstelle bereitzustellen, über die gemäß einem Benutzereingabesignal eingestellt wird, ob mindestens eines von der Modellierung des Fötus und der Schätzung eines Ergebnisses der Modellierung des Fötus automatisch oder manuell durchgeführt werden soll.

## Revendications

1. Procédé de mesure d'une biométrique d'un sujet, le procédé comprenant :
la réception d'une image de l'objet (S301) ;
la modélisation de l'objet de telle sorte qu'au moins une partie de l'objet soit identifiée (S303) ; et
la mesure d'une biométrique de l'objet, d'après un résultat de modélisation de l'objet (S305) ; dans lequel l'objet est un foetus ;
dans lequel :
la biométrique est une longueur vertex-coccyx (CRL) du foetus et la modélisation de l'objet comprend la modélisation de l'objet de telle sorte qu'un corps (20) et une tête (10) de l'objet soient identifiés (S503),
et après la modélisation de l'objet, le procédé comprenant en outre :
la détection d'au moins un point caractéristique sur la tête du foetus ;
l'établissement d'un axe central (21) par l'utilisation de l'au moins un point caractéristique, et l'affichage de l'axe central ;
la mesure d'un angle (51) entre le corps et la tête du foetus par rapport à l'axe central (S510) ;
le fait de déterminer si l'angle entre ou non dans une plage normale (S513) ; et
la mesure d'une longueur vertex-coccyx (CRL) du foetus,
dans lequel la mesure de la CRL comprend :
si l'angle entre dans la plage normale, la mesure de la CRL du foetus d'après le résultat de modélisation du foetus (S523) ;
si l'angle n'entre pas dans la plage normale, l'estimation d'un modèle du foetus correspondant à un cas où l'angle entre dans la plage normale (S515), et la mesure de la CRL du foetus, d'après le résultat de modélisation estimé (S517) ; et
l'affichage du résultat de modélisation estimé et de la CRL mesurée.

2. Procédé selon la revendication 1, dans lequel la mesure de la biométrique de l'objet comprend :
la détection d'une région d'intérêt, ROI, (S607) pour mesurer la biométrique, d'après le résultat de modélisation de l'objet ; et
la mesure de la biométrique dans la ROI (S615).

3. Procédé selon la revendication 2, dans lequel la détection de la ROI comprend :
l'affichage de la ROI détectée pour qu'elle soit différenciée des autres parties de l'objet ;
l'affichage d'une région pour mesurer la biométrique, dans la ROI ; et
la modification de la ROI ou de la région pour mesurer la biométrique (S610), selon un signal d'entrée utilisateur.

4. Procédé selon la revendication 1, dans lequel la mesure de la biométrique comprend :
la mesure d'une longueur vertex-coccyx (CRL) du foetus (S1223) ;
la mesure d'au moins l'une parmi une translucidité nucale (NT) et une translucidité intracrânienne (IT) du foetus (S1320) ;
le calcul d'une différence relative entre la CRL et soit la NT soit l'IT (S1323) ; et
l'affichage des CRL, NT, et IT mesurées (S1330).

5. Appareil terminal pour mesurer une biométrique d'un foetus, l'appareil terminal comprenant :
une unité de stockage pour stocker une image du foetus (110) ; et
une unité de commande (120) comprenant :
un module de modélisation pour modéliser le foetus de telle sorte qu'au moins une partie du foetus soit identifiée dans l'image de l'objet ; et
un module de mesure pour mesurer une biométrique du foetus, d'après un résultat de modélisation du foetus ; dans lequel :
le module de modélisation est agencé pour modéliser le foetus de telle sorte qu'un corps et une tête du foetus soient identifiés et pour détecter au moins un point caractéristique sur la tête du foetus, et pour établir un axe central par l'utilisation de l'au moins un point caractéristique, l'unité de stockage est adaptée pour stocker des données biométriques incluant des informations à propos d'une plage normale d'au moins une biométrique,
le module de mesure est agencé pour mesurer un angle entre le corps et la tête du foetus, pour déterminer si l'angle entre ou non dans une plage normale, et pour mesurer une longueur vertex-coccyx (CRL) du foetus, et
l'appareil terminal comprend en outre une unité de sortie pour fournir en sortie un résultat d'estimation d'un résultat de modélisation du foetus lorsque l'angle entre dans une plage normale et un résultat de mesure de la CRL du foetus ;
si l'angle entre dans la plage normale, le module de mesure mesure la CRL du fétus d'après le résultat de modélisation du foetus, et
si l'angle n'entre pas dans la plage normale, le module de mesure estime un modèle du foetus correspondant à un cas où l'angle entre dans la plage normale, et mesure la CRL du foetus d'après le résultat de modélisation estimé.

6. Appareil terminal selon la revendication 5, dans lequel le module de mesure est agencé pour détecter une région d'intérêt (ROI) pour mesurer la biométrique, d'après le résultat de modélisation de l'objet, et pour mesurer la biométrique dans la ROI, et
l'appareil terminal comprend en outre une unité de sortie pour fournir en sortie la ROI détectée pour qu'elle soit différenciée des autres parties de l'objet, et fournir en sortie une région pour mesurer la biométrique dans la ROI.

7. Appareil terminal selon la revendication 5, dans lequel l'unité de commande comprend en outre un module de calcul pour calculer un taux d'erreur entre un résultat de mesure de la biométrique à nouveau et la biométrique mesurée à l'aide du résultat de modélisation de l'objet.

8. Appareil terminal selon la revendication 5, dans lequel le module de mesure est adapté pour mesurer la CRL du foetus, et mesure au moins l'une parmi une translucidité nucale (NT) et une translucidité intracrânienne (IT) du foetus, et
l'unité de commande comprend en outre un module de calcul pour calculer une différence relative entre la CRL et soit la NT soit l'IT.

9. Appareil terminal selon la revendication 5, dans lequel l'unité de commande est adaptée pour fournir une interface utilisateur via laquelle on établit ou non s'il faut réaliser automatiquement ou manuellement au moins l'une parmi la modélisation du foetus et l'estimation d'un résultat de modélisation du foetus selon un signal d'entrée utilisateur.
